# EUROPEAN PATENT SPECIFICATION

(11) **EP 4 106 823 B1**
(45) Date of publication and mention of the grant of the patent: **10.12.2025**
(21) Application number: 20919768.0
(22) Date of filing: 18.02.2020
(51) Int. Cl.: A61L 15/16, A61F 13/15

(54) **THREE-DIMENSIONAL SUBSTRATE AND ABSORBENT ARTICLES COMPRISING THE SAME**
DREIDIMENSIONALES SUBSTRAT UND DIESES UMFASSENDE ABSORBIERENDE ARTIKEL
SUBSTRAT TRIDIMENSIONNEL ET ARTICLES ABSORBANTS LE COMPRENANT

(43) Date of publication of application: 28.12.2022
(73) Proprietor: The Procter & Gamble Company, Cincinnati, OH 45202 (US)
(72) Inventor: ERDEM, Gueltekin, Beijing 101312 (CN); YUAN, Yi, Beijing 101312 (CN)
(74) Representative: P&G Patent Germany
(86) International application number: PCT/CN2020/075656
(87) International publication number: WO 2021/163868

(56) References cited:
- EP-A2- 0 962 208
- WO-A1-01/06974
- WO-A1-01/06974
- WO-A1-2019/076288
- CN-U- 209 770 694
- JP-A- 2000 354 601
- JP-A- 2012 095 936
- JP-A- 2016 097 107
- US-A1- 2019 240 084

## Description

### FIELD OF THE INVENTION

The invention is for a three-dimensional substrate and an absorbent article containing the same such as a baby diaper, a training pant, a feminine hygiene sanitary napkin or an adult incontinence product.

### BACKGROUND OF THE INVENTION

Absorbent articles for personal hygiene, such as disposable diapers for infants, training pants for toddlers, adult incontinence undergarments and/or sanitary napkins are designed to absorb and contain body exudates, in particular large quantities of urine, runny bowel movement (BM) and/or menses.

These absorbent articles comprise several layers providing different functions. A liquid permeable topsheet is disposed closest to the wearer's skin and should be capable of quickly absorbing the excreted fluid. A backsheet is disposed on the opposed, garment-facing side of the article. Many absorbent articles in the market further comprises a nonwoven outermost layer forming at least part of a garment-facing surface of an absorbent article. Other components of absorbent articles are well known, and include in particular an absorbent core disposed between the topsheet and the backsheet to absorb and retain the excreted fluids.

Three-dimensional substrates comprising at least two layers and three-dimensional elements and hollow spaces between the two layers may provide improved fluid handling properties, and improved sensory feels such as skin softness, and cushion feel, etc. Three-dimensional substrates may also provide craftmanship perception. Use of such three-dimensional substrate as a topsheet in an absorbent article has been disclosed, for example, in US2014/0121625A1. While the three-dimensional substrates have merits, they have inherent problems caused by the structure of three-dimensional substrates having a plurality of hollow areas between constituting layers. The three-dimensional substrates are subject to easy deformation, worn-out and integrity issues when the substrate is used as a component of products which directly interacts with skin or a garment. Another problem is a delamination issue. When the three-dimensional substrates are integrated into a product, the three-dimensional substrate is cut into certain dimensions which may render some of hollow areas are cut through and exposed. As those hollow areas exposed are unbonded or loosely bonded areas in the substrate, they become spots rendering delamination of layers constituting the three-dimensional substrate. This may be more serious problem when the substrate is used as a component having an exposed longitudinal edge and/or transversal edge such as a topsheet or an outer most layer in an absorbent article.

WO2019076288 relates to topsheets for use with absorbent articles comprising a first layer and a second layer. The first layer is a spunlace nonwoven and comprises at least 15% by weight of natural fibers, a plurality of protrusions and a plurality of apertures. The first layer and the second layer are in contact with each other between the majority of the protrusions.

It is desirable to provide three-dimensional nonwovens achieving sufficient integrity without compromising targeted benefits such as soft and cushiony feel.

It is also desirable to provide absorbent articles comprising a three-dimensional substrate having characteristics of being soft and cushiony, and a structural strength to avoid delamination in the three-dimensional substrate.

### SUMMARY OF THE INVENTION

The invention provides a three-dimensional substrate and an absorbent article as defined in the claims.

The article is illustrated in the Figures as a taped diaper. For ease of discussion, the absorbent article and the three-dimensional substrate will be discussed with reference to the numerals referred to in these Figures. The Figures and detailed description should however not be considered limiting the scope of the claims, unless explicitly indicated otherwise, and the invention disclosed herein is also used in a wide variety of absorbent article forms.

### BRIEF DESCRIPTION OF THE DRAWINGS

Fig. 1A is a schematic cross-sectional view of an exemplary substrate of the present invention.
Fig. 1B is a schematic cross-sectional view of an alternative substrate of the present invention.
Fig. 2 is a schematic illustration of a top view of an exemplary substrate of the present invention.
Fig. 3 is a schematic illustration of a top view of another exemplary substrate of the present invention.
Fig. 4 is a schematic illustration of an exemplary process and apparatus for making a substrate of Fig. 1B.
Fig. 5 is a schematic illustration of another process and apparatus for making a substrate of Fig. 1B.
Fig. 6 is a simplified front view of an apparatus in Fig. 5 showing arrangement of roll B and nip rolls E.
Fig. 7 is a simplified front view of another apparatus in Fig. 5 showing arrangement of roll B and nip rolls E.
Fig. 8 is a schematic plan view of an exemplary absorbent article according to the present invention.
Fig. 9 is a lateral cross-section view along 2-2 of the absorbent article of Fig. 8.
Fig. 10 is a photograph of a three-dimensional substrate of the present invention.

### DETAILED DESCRIPTION OF THE INVENTION

Various non-limiting forms of the present disclosure will now be described to provide an overall understanding of the principles of the structure, function, manufacture, and use of an absorbent article according to the present invention. One or more examples of these non-limiting embodiments are illustrated in the accompanying drawings. The features illustrated or described in connection with one non-limiting form may be combined with the features of other non-limiting forms. Such modifications and variations are intended to be included within the scope of the present disclosure.

### Definitions of terms

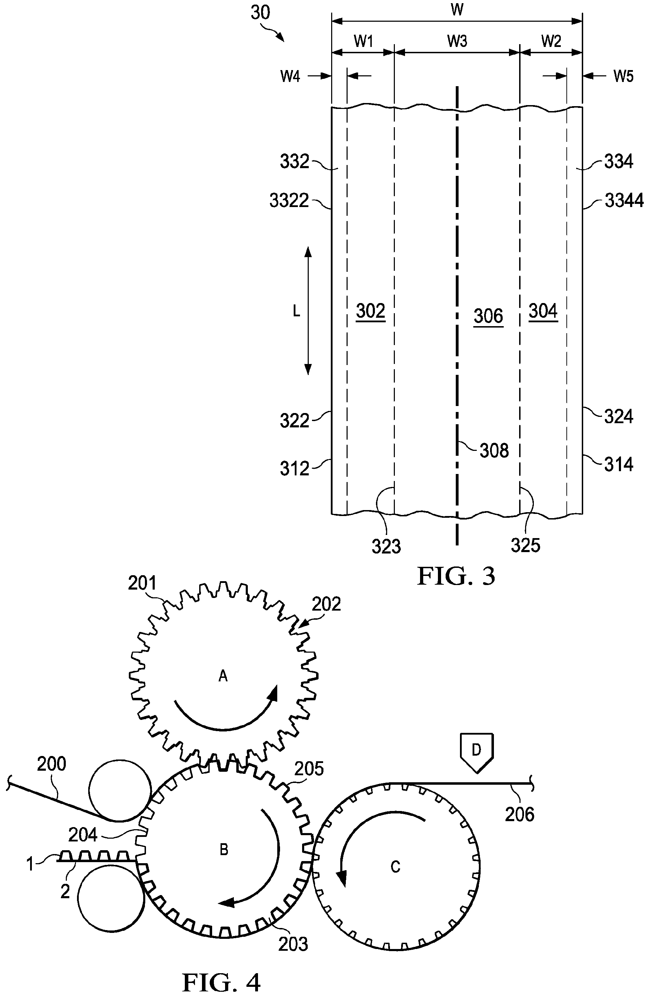

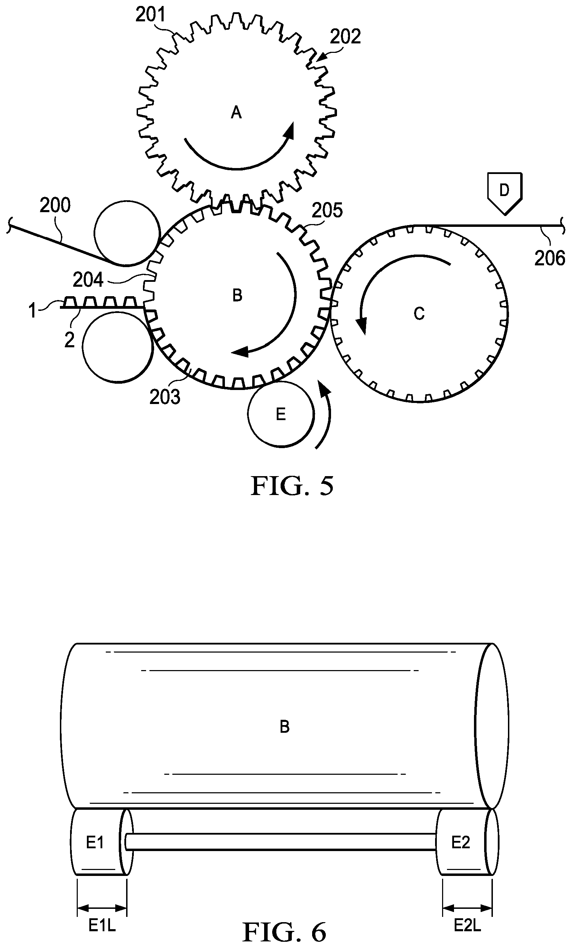

The term "absorbent article" as used herein refers to disposable products such as taped diapers, diapers having a closed waist opening (pants), feminine hygiene sanitary napkins and the like, which are placed against or in proximity to the body of the wearer to absorb and contain bodily exudates such as urine, feces and menses discharged from the body. Typical absorbent articles comprise a topsheet, a backsheet, an absorbent core, an acquisition layer and other components. A liquid permeable topsheet forms at least a portion of the wearer-facing side of the article, and a backsheet forms at least a portion, and typically the whole, of the garment-side of the article. The articles may be provided with fastening elements, such as tapes (taped diapers) or may be provided already pre-formed with a waist opening and a pair of leg openings as in an underwear (pant diapers). The absorbent articles may be for use with babies, infants, women or incontinent adults. Typical features of absorbent articles are discussed further below, and in relation with the illustrated taped diaper in Figs. 4 and 5, which is of course for illustration purpose only and not limiting the scope of the inventions, unless specifically indicated otherwise.

"Machine direction" (MD) means the direction of material flow through a process. In addition, relative placement and movement of material can be described as flowing in the machine direction through a process from upstream in the process to downstream in the process. When a material is removed from a finished product, "machine direction" intends to mean a longitudinal direction of the product. "Cross direction" (CD) means a direction that is generally perpendicular to the machine direction. When a material is removed from a finished product, "cross direction" intends to mean a transversal direction of the product. "Z-direction," with respect to a web, means generally orthogonal or perpendicular to the plane approximated by the web along the machine and cross direction dimensions.

The term "nonwoven" as used herein refers to a manufactured material, web, sheet or batt of directionally or randomly oriented fibers, bonded by friction, and/or cohesion and/or adhesion, excluding paper and products which are woven, knitted, tufted, stitch-bonded, incorporating binding yarns or filaments, or felted by wet milling, whether or not additionally needled. The fibers may be of natural or man-made origin. The fibers may be staple or continuous filaments or be formed in situ. The porous, fibrous structure of a nonwoven may be configured to be liquid permeable or impermeable, as desired.

"Comprise," "comprising," and "comprises" are open ended terms, each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essential of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "advantageously" and the likes also qualify features which are not intended to limit the scope of the claims unless specifically indicated to do so.

### Three-dimensional substrate

The three-dimensional substrate (herein also more simply referred to as "the substrate") disclosed herein comprises a first layer and a second layer intermittently bonded to the first layer. While the first layer and the second layer will be discussed individually in greater details in dedicated sections further below, this section will briefly discuss the substrate comprising these two layers, as a whole.

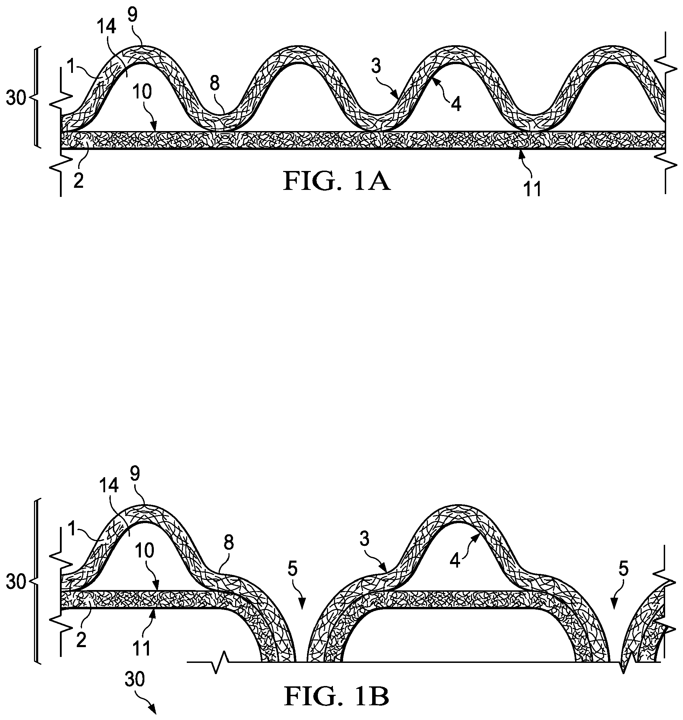

Figs. 1A and 1B schematically show cross-sections of two exemplary substrates 30 according to the invention. The substrate 30 comprises a first layer 1 and a second layer 2. The first layer 1 and the second layer 2 are disposed in a face to face relationship in the substrate 30. The first layer 1 comprises a first surface 3 and a second surface 4. The second layer 2 comprises a first surface 10 and a second surface 11. The first surface 10 of the second layer 2 faces the second surface 4 of the first layer 1. The first surface 3 of the first layer 1 and the first surface 10 of the second layer 2 are typically facing outwardly (externally), towards the wearer-facing side of the article, while the second surface 4 of the first layer 1 and the second surface 11 of the second layer 2 are typically facing inwardly (internally), when the substrate is incorporated in an absorbent article. The first layer 1 comprises a plurality of protrusions 9 which are unbonded to the second layer 2, so that the first layer 1 is intermittently bonded to the second layer 2. The protrusions 9 provide a three-dimensional profile to the first layer 1 and more generally to the substrate 30 as a whole.

The first layer 1 and the second layer 2 may be generally contiguous in the horizontal plane, but it is not excluded that the second layer may be wider or longer than the first layer. In this example, the first layer may have a smaller area disposed in the central region of the article relative to the second layer. The protrusions may also present only in a selected area of the substrate that is smaller than the overall surface of the substrate.

The first layer 1 and the second layer 2 forming the substrate 30 are intermittently bonded by technologies known in the art. For example, the first layer 1 and the second layer 2 can be laminated by either adhesive or thermally bonded means, where thermal bonding includes but is not restricted to technologies such as ultrasonic bonding, cold pressure bonding, and hot pressure bonding. When the first layer comprises a relatively high amount of natural fibers, bonding the first and second layer to each other using heat may be difficult as the natural fibers do not become tacky or melt upon exposure to heat. The first layer 1 is thus attached to the second layer 2 in bonding areas by hot-melt adhesive.

The substrate 30 according to the present invention, referring to Fig. 1B, may further comprise apertures 5. The apertures 5 in the substrate can enhance the overall air flow and breathability of the absorbent article.

In some embodiments referring to Figs. 1A and 1B, in a substrate perspective, the protrusions can have hollow space and thus comprise an inside void volume 14 which is the portion of the protrusion which does not comprise any fibers, or at least very little fibers. The presence of protrusions can typically improve the softness of the substrate, and thus of the garment-facing side of the article on which it is disposed. The void volume 14 may have a width WD, which is the maximum interior width measured between two side walls of the inner protrusion (or which is the maximum diameter of the side wall of the inner protrusion when the distal portion has a substantially circular shape). The width WD of the protrusions 9 may for example range from 0.5 mm to 15 mm or from 0.5 mm to 10 mm or from 0.5 mm to 5 mm or from 0.5 mm to 3 mm. Measurements of the dimensions of the width of the height of the protrusions 9 can be made on a photomicrograph.

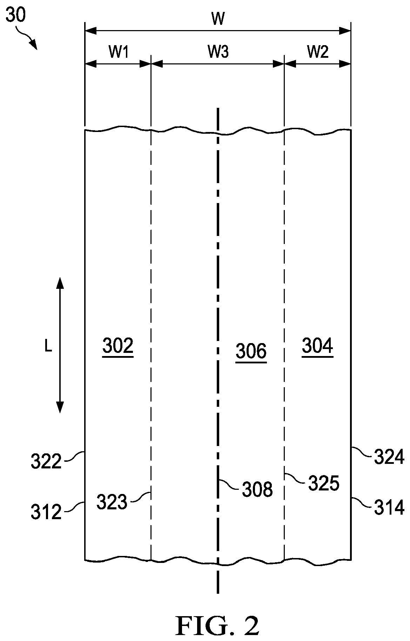

Referring to Fig. 2, looking down on a top view of a three-dimensional substrate 30 of the present invention, the substrate 30 comprises a first longitudinal edge 312, a second longitudinal edge 314, and a longitudinal axis 308, and comprises a first region 302 longitudinally extending along the first longitudinal edge 312, a second region 304 longitudinally extending along the second longitudinal edge 314, and a third region 306 located between the first region 302 and second region 304 about a transversal direction. The first region 302 has a first peel force, the second region 304 has a second peel force, and the third region 306 has a third peel force, as measured according to Peel Force Test, and at least one of the first peel force and the second peel force is higher than the third peel force.

In some embodiments, the first peel force and/or the second peel force is no less than 0.15 N/50.8mm, or no less than 0.20N/50.8mm, or no less than 0.30N/50.8mm or no less than 0.35 N/50.8mm. In the embodiments, the third peel force may be lower than about 4.5N/50.8mm, or lower than about 3.5N/50.8mm, or lower than about 2.5N/50.8mm, or lower than about 1.5N/50.8mm in view of avoiding deteriorating softness and cushiony feel of the substrate.

Still referring to referring to Fig. 2, the substrate 30 comprises the substrate 30 comprises a first longitudinal edge 312, a second longitudinal edge 314, and a longitudinal axis 308, and comprises a first region 302 longitudinally extending along the first longitudinal edge 312 and having a first peel force, a second region 304 longitudinally extending along the second longitudinal edge 314 and having a second peel force, and a third region 306 located between the first region 302 and second region 304 about a transversal direction and having a third peel force. The first peel force and the second peel force may be no less than about 0.15 N/50.8mm, or no less than 0.20N/50.8mm, or no less than 0.30N/50.8mm or no less than 0.35 N/50.8mm. The third peel force islower than at least one of the first peel force and the second peel force. The third peel force may be substantially the same as the first peel force or the second feel force.

Still referring to Fig. 2, the first region 302 may have a first side edge 322 consistent with the first longitudinal edge 312 and a second side edge 323, and the second region 304 may have a first side edge 324 consistent with the second longitudinal edge 314 and a second side edge 325. The substrate 30 of the present invention has a transversal directional width of W perpendicular to a longitudinal direction L, and the first region 302, the second region 304, and the third region 306 have widths of W1, W2 and W3, respectively. The widths W1, W2 and W3 may be determined considering usage and application of the substrate.

In some embodiments, the third region 306 may have a width no less than about 50 mm, or no less than about 60mm, or no less than about 80mm, or no less than 100mmThe third region has a width W3 at least about 30%, or at least about 40% or at least about 50% of the substrate width W. In some embodiment, the third region includes the longitudinal axis 308.

To resolve a delamination problem, peel force between the first layer and the second layer in the substrate may increase. However, increase of peel force in the entire substrate may deteriorate softness and cushiony feel of the substrate, so that it may not be favorable especially when the substrate is used as a component of products which directly interacts with wearer's or care-giver's skin such as a topsheet or outer cover sheet in absorbent articles.

In the first invention disclosed herein, peel force difference in at least one the first and the second regions over the third region may be achieved by known and suitable methods. As one example, higher levels of heat or pressure can be applied to at least part of the first and/or the second regions over the third region using embossing or bonding process to bond the first layer and the second layer, for example. As another example, embossing or bonding areas can be varied in at least part of in the first and/or the second regions over the third region. As another example, applying different type of adhesive and/or different amount of adhesive on at least part of the first and/or at least part of the second regions than the third region.

Referring to Fig. 3, the first region 302 may comprise a first area 332, and the second region 304 may comprise a second area 334. The first area 332 and the second area 334 each is an area which contributes to increase peel forces in the first region 302 and the second region 304. The first layer and the second layer forming the substrate 30 in the first area 332 or the second area 332 may be bonded by technologies known in the art such as adhesive and thermally bonded means to have a higher peel force than a predetermined value.

The first area 332 may have a longitudinal edge 3322, and the second area 334 may have a longitudinal edge 3344. The longitudinal edges 3322 and 3344 may be at least partially consistent with the first longitudinal edge 312 and the second longitudinal edge 314 of a substrate 30. The longitudinal edges 3322 and 3344 may be substantially consistent with a first longitudinal edge 312 and the second longitudinal edge 314 of a substrate 30 as exemplified in Fig. 3. In another embodiment, a longitudinal edge 3322 of the first area 332 may locate between a first side edge 322 and a second side edge 323 of a first region 302 and/or a longitudinal edge 3344 of a second area 334 may locate between a first side edge 324 and a second side edge 325 of a second region 304. Although the first area 332 and the second area 334 are illustrated as being rectangular in Fig. 3, the first area 332 and the second area 334 may also have any other suitable shapes. Each of the first area 332 and the second area 334 may comprise a single continuous area. Each of the first area 332 and the second area 334 may comprise multiple discrete sub-areas. The first area 332 and the second area 334 may be symmetrical, mirror-image symmetrical, or asymmetrical to each other. In some embodiments, the substrate 30 is more compressed in at least one of the first area 332 and the second area 334 that other areas. In other embodiments, the first layer and the second layer are substantially bonded to each other in at least one of the first area 332 and the second area 334. In other embodiments, at least one of the first area 332 and the area 334 comprises a higher basis weight of adhesive than the third region 306.

The first layer 1 and the second layer 2 forming the substrate 30 are intermittently bonded by technologies known in the art. For example, the first layer 1 and the second layer 2 can be laminated by either adhesive or thermally bonded means, where thermal bonding includes but is not restricted to technologies such as ultrasonic bonding, cold pressure bonding, and hot pressure bonding. When the first layer comprises a relatively high amount of natural fibers, bonding the first and second layer to each other using heat may be difficult as the natural fibers do not become tacky or melt upon exposure to heat. The first layer 1 is thus attached to the second layer 2 in bonding areas by hot-melt adhesive.

The substrate including the first layer and second layer may typically have a basis weight from 15 g/m² to 80 g/m², or from 15 g/m² to 60 g/m², or from 20 g/m² to 50 g/m². The first layer may have a basis weight of from 10 g/m² to 50 g/m², or from 15 g/m² to 40 g/m². The second layer may have a basis weight of from 5 g/m² to 50 g/m², or from 7 g/m² to 30 g/m², or from 7 g/m² to 20 g/m². The second layer may have a basis weight that is at least 5 g/m² lower, or at least 10 g/m² lower, than the basis weight of the first layer. It has been found that such relatively low basis weight layers are sufficient to facilitate stabilization of the three-dimensional configuration of the first layer. The substrate typically comprises these two layers only, but it is not excluded that a third or more layers may also be present in the substrate.

The substrate may be in a close contact with adjacent layers of the absorbent article.

### Protrusions

Referring to Figs. 1A and 1B, cross-sectional views, a three-dimensional substrate 30 disclosed herein comprises a plurality of protrusions. The plurality of protrusions 9 imparts a three-dimensional shape to the first layer and the substrate.

Viewed from a cross-sectional view as in Figs. 1A and 1B, i.e. in a Z-direction, the protrusions may have any suitable shapes, including but not limited to: cylindrical, bulbous-shaped, conical-shaped, mushroom shaped and heart-shaped. Viewed from above, the majority of the protrusions may have any suitable shapes, including but not limited to: circular, diamond-shaped, round diamond-shaped, U.S. football-shaped, oval-shaped, clover-shaped, triangular-shaped, tear-drop shaped and elliptical-shaped protrusions. The protrusions may have a Z-directional height in the range from about 0.1 mm to about 6.0 mm, or from about 0.3 mm to about 4.0 mm, or from about 0.5 mm to about 3.0 mm, measured from the land areas 8 to the top of the protrusion.

The protrusions 9 may be uniformly distributed on the first surface 3 of the first layer 1. The protrusions 9 may be provided throughout the complete surface of the first layer 1 or may only be provided in a portion of the first layer 1. Two or more adjacent protrusions 9 are separated by one or more land areas 8 and optionally one or more apertures 5. The protrusions 9 may be surrounded by a plurality of land areas 8 and/or a plurality of apertures 5. The land areas 8 may be substantially flat areas. In some embodiments, the land areas 8 are flat areas. The land areas 8 may fully surround the protrusions 9. The optional apertures 5 may be located between the protrusions and will be discussed further below. The protrusions may extend upwardly from land areas 8 that form a base and have an opposed distal portion from the land areas 8 forming a peak. The base of the protrusions 9, where each protrusion starts to protrude outwardly from the land areas 8, define a perimeter, which for circular protrusions is the circumference.

In some embodiments referring to Figs. 1A and 1B, the protrusions are formed on the first layer 1, and the second layer 2 may be free of protrusions. The area of the second layer which coincides with the protrusions of the first layer, may be substantially flat.

The first layer 1 and the second layer 2 may be joined with each other at the land areas 8, at least partially, between the protrusions 9 and/or at the apertures 5. The first layer 1 and the second layer 2 are typically not in contact in the areas of the protrusions 9.

### First layer

The substrate disclosed herein, referring to Figs. 1A and 1B, comprises a first layer 1, which is typically disposed more outwardly than the second layer 2, relative to an absorbent article to form at least part of an inter most layer or an outermost layer of the absorbent article when the substrate is incorporated in an absorbent article.

The first layer is a nonwoven web of natural fibers, synthetic fibers or a combination of natural and synthetic fibers. Several examples of nonwoven materials suitable for use as a first layer include, but are not limited to: spunbonded nonwovens; carded nonwovens; air through bonded carded nonwovens; spunlace nonwovens; needle punched nonwovens and nonwovens with relatively specific properties to be able to be readily deformed.

The first layer may comprise synthetic fibers, or mixed with the natural fibers. Synthetic fibers may be selected from the group consisting of polypropylene, polyethylene, polyester, polyethylene terephthalate, polybutylene terephthalate, polyamide, polylactic acid, and combinations thereof. The first layer may comprise heat fusible fibers, which may be mixed with natural fibers in the first layer of the topsheet. The term "heat fusible fibers" means fibers that when they are heated at a certain temperature, the fibers can fusion bond to other fibers that comprise the same material or different material from the heat fusible fibers.

The first layer may comprise natural fibers, such as cotton fibers and rayon fibers, to improve the softness of the substrate, as well as to increase the amount of biodegradable material used. Using natural fibers for layers on the wearer-facing side of absorbent articles is generally desired. The first layer 1 may thus comprise at least 15% by weight, or at least 30% by weight, or at least 50% by weight, or at least 60% by weight, or at least 75% by weight, or at least 80% or at least 95% by weight of natural fibers, such as cotton fibers, by weight of the first layer. The first layer may also be made of 99% to 100% by weight of natural fibers, such as cotton fibers, by weight of the first layer. Natural fibers may be selected from the group consisting of wheat straw fibers, rice straw fibers, flax fibers, bamboo fibers, cotton fibers, jute fibers, hemp fibers, sisal fibers, bagasse fibers, hesperaloe fibers, miscanthus, marine or fresh water algae/seaweeds and combinations thereof.

The first layer may be hydrophilic or hydrophobic. In order to have a hydrophobic first layer, a hydrophobic treatment may be applied to the first layer. The hydrophobic treatment may be based on synthetic material, at least to some extent, derived from natural sources. The hydrophobic treatment may be based on a natural compound, such as selected from the group consisting of natural oil, butters or waxes and combination thereof. Some examples, but not limited to, are cotton seed oil, Coconut oil, Avocado oil, Jojoba oil, Castor-seed oil, Soybean oil, Almond oil, Lanolin, Olive oil, Sunflower seed oil, Eucalyptus oil, Shea butter, Cocoa butter, Murumuru butter, Almond butter, Avocado butter, Aloe butter, Mango butter, Beeswax, Soy wax, Candelilla wax, Rice-bran wax, Coconut wax.

### Second layer

The substrate disclosed herein, referring to Figs. 1A and 1B, comprises a second layer 2 in a face to face relationship with the first layer 1, as described above. The second layer 2 is a nonwoven web of natural fibers, synthetic fibers or a combination of natural and synthetic fibers. Alternatively, the second layer may be a polymeric film as is commonly used in backsheet. Typically, the polymer film may be breathable or not, but is liquid impermeable.

The list of synthetic fibers and of natural fibers corresponds to the list disclosed above for the first layer. Typically, the synthetic fibers are selected from the group consisting of polypropylene, polyethylene, polyester, polyethylene terephthalate, polybutylene terephthalate, polyamide, polylactic acid, and combinations thereof. The synthetic fibers may be single component fibers, multi-component fibers such as bicomponent fibers and combinations thereof. The fibers may have any suitable deniers or denier ranges and/or fiber lengths or fiber length ranges. The fibers in the nonwoven web can then be bonded via spunlacing processes, hydroentangling, calendar bonding, through-air bonding and resin bonding. The second layer may typically have a basis weight of from 5 g/m² to 50 g/m², or from 7 g/m² to 30 g/m², or from 7 g/m² to 20 g/m².

Several examples of nonwoven materials suitable for use as a second layer include, but are not limited to, those disclosed above with respect to the first layer.

### Apertures

The substrate of the present invention optionally comprises a plurality of apertures. In some embodiments, the first layer of the substrate comprises a plurality of apertures 5. In the embodiments, the second layer of the substrate may have a plurality of apertures 5 at least partially, or completely aligned with the apertures 5 of the first layer 1. The apertures 5 of the first layer 1 and of the second layer 2 may thus be the same apertures. The plurality of apertures 5 of the second layer 2 may have at least partially the same width and/or length as the apertures 5 of the first layer 1.

In some embodiments, as illustrated in Fig. 1B, the substrate 30 may comprise a plurality of apertures 5 extending through the first layer 1 and the second layer 2.

The apertures 5 may be typically regularly aligned according to a desired pattern.

The plurality of apertures 5 may be uniformly distributed on the first layer 1. To ensure material stability, the smallest distance between the majority of the apertures regardless of their particular shape and size may be at least 0.3 mm, or at least 1.0 mm. This distance is measured center-to-center on the first surface of the first layer of the topsheet.

### Method of Making the Three-dimensional Substrate

The three-dimensional substrate (with or without apertures) may be industrially produced at high speed by any known and suitable methods, for example as described in WO2017/156200 (Orr et al., P&G) and PCT/CN2018/110397, filed on Oct. 16, 2018 (Erdem et al, P&G). Fig. 4 is a schematic illustration of one example process for forming protrusions in the first layer and bonding this layer to a second layer, which is briefly discussed below. More details of this process, including photographs of the embossing rolls, can be found in PCT/CN2018/110397.

Referring to Fig. 4, a first layer material 200 may go through a pair of rolls named A and B. The first layer material 200 is to form the first layer 1 of the substrate.

The speed of the roll A and B may be from 5 to 600 meters/minute. The temperature range of the roll A may be from 40 to 200°C. The temperature range of the roll B may be from 30 to 200°C. The roll A may comprise a plurality of protrusions 201 extending radially outwardly from the roll A. The roll A may also comprise a plurality of recesses 202 formed in a radial outer surface of the roll A. The depth of the recesses 202 of the roll A may be from 0.5 to 10 mm, the height of the protrusions 201 of the roll A may be from 0.5 to 9 mm. The roll B may comprise a plurality of protrusions 203 extending radially outwardly from the roll B. The roll B may also comprise a plurality of recesses 204 formed in a radial outer surface of the roll B. The distal end of the plurality of protrusions 203 of the roll B may have the shape of a pin 205.

The protrusions 201 on the roll A may have a different size, shape, height, area, width and/or dimension than the protrusions 203 on the roll B. The recesses 202 formed in the roll A may have a different size, shape, height, area, width, and/or dimension than the recesses 204 formed in the roll B. The recesses 202 in the roll A may be configured to at least partially receive the protrusions 203 of the roll B, thereby creating the protrusions in the first layer material 200. The roll A may comprise a plurality of holes in the recesses area in order to receive the shape of pin 205 of the protrusions 203 of the roll B. Therefore, a plurality of apertures 5 are formed in the first layer material 200 between each two adjacent protrusions of the first layer material 200. The first layer material 200, after going through the roll A and the roll B may comprise a plurality of protrusions 9 and a plurality of apertures 5 between each two adjacent protrusions.

A second layer material 206 may be brought by a roll C. Adhesive such as hot-melt adhesive can be added on the first surface of the second layer material 206 by an equipment D before the second layer material 206 is in contact with the first layer material 200. The hot-melt adhesive can be advantageously uniformly sprayed on the second layer material at the basis weight indicated previously, for example 1-10 g/m². The roll C may comprise a plurality of holes in order to receive the shape of pin 205 of the protrusions 203 of the roll B. The second layer material 206 may pass through the roll C and the roll B and contact with the first layer material 200 at the protrusions 203 of the roll B. As the protrusions 203 of the roll B may have the shape of a pin, a plurality of apertures may be created also on the second layer material 206. The apertures in the second layer material 206 may in this way be aligned with the apertures in the first layer material 200. Pressure is applied in the land areas of the first layer and the second layer so that the adhesive forms bonds between the first and second layers to form the substrate. At the end of this process, a substrate material as illustrated in Fig. 1B comprising a three-dimensional, apertured first layer 1 in contact with a second layer 2 between the protrusions 9 of the first layer is obtained.

Penetration of at least part of fibers of the first layer into the second layer at the apertures improves the integrity of the substrate by reducing the risk of delamination of the first and second layer. Fiber penetration creates "anchor points" which stabilizes the substrate of first and second layer, even if the contact area between first and second layer is reduced as only the first layer has protrusions. The anchor points also reduce the risk of fiber fuzzing.

A substrate 30 for the invention but without apertures may be more easily made by first forming a three-dimensional first layer, typically by engaging a first layer material between a first and second forming members and mechanically deforming the material to form a first layer having a three-dimensional shape. Such methods are well known, and for example are described in WO2017/156200 and WO2017/156203, and PCT/CN2018/110397. The adhesive is then applied, e.g. by spraying, on the second layer material and the first layer material is bonded to the second layer material by applying pressure in the land areas 8, but without aperturing, to form the dual layer substrate of the invention.

In addition to using adhesive or alternatively, the first layer may be attached to the second layer in the bonding areas by embossing or by pin bonding. The term "embossing" means creating bonding points between the first layer and the second layer by heat or pressure for example.

Higher peel forces in at least one the first and the second regions over the third region may be achieved by known and suitable methods. The process in Fig. 5 is similar to the process shown in Fig. 4 except having at least one additional nip roll E engaging with roll B to provide higher compression on at least part of the first region 302 and/or at least part of the second region 303, after a second layer material 206 is brought by roll C. After the first layer material 200 and the second layer material 206 are combined by passing through the roll B and roll C, the combined material may pass through the roll B and at least one nip roll E by which additional pressure is applied in the areas of the substrate where the roll B and the nip roll E are engaged, so that the substrate has at least one of two side regions (a first region and a second region) having a higher peel force than a third region located between the first and second regions. Fig. 6 is an example apparatus suitable for the process shown in Fig. 5 comprising a plurality of nip rolls, E1 and E2 (collectively, "E"). Referring to Fig. 6 and Fig. 3, rip rolls E1 and E2 contacting the roll B provide higher compression on a first area 332 and a second area 334 in a three-dimensional substrate 30 where the rip rolls E1 and E2 contact the three-dimensional substrate 30. The length E1L of nip roll E1, and the length E2L of nib roll E2 may determine a width W4 of the first area 332, and a width W5 of the second area 334, respectively. In one embodiment, a longitudinal edge 3322 of a first area 332 may be at least partially consistent with a first longitudinal edge 312 of a substrate 30. The longitudinal edge 3344 of a second area 334 may be substantially consistent with the first longitudinal edge 312 of the substrate 30 as exemplified in Fig. 3. In another embodiment, a longitudinal edge 3322 of a first area 332 may locate between a first longitudinal edge 312 of a substrate 30 and a second side edge 323 of a first region 302 and/or a longitudinal edge 3344 of a second area 334 may locate between a second longitudinal edge 314 of the substrate 30 and a second side edge 325 of a second region 304. The same process can be used to produce a three-dimensional substrate 30 having a first region 302 and a second region 304 having peel force higher than about 0.15N/50.8m.

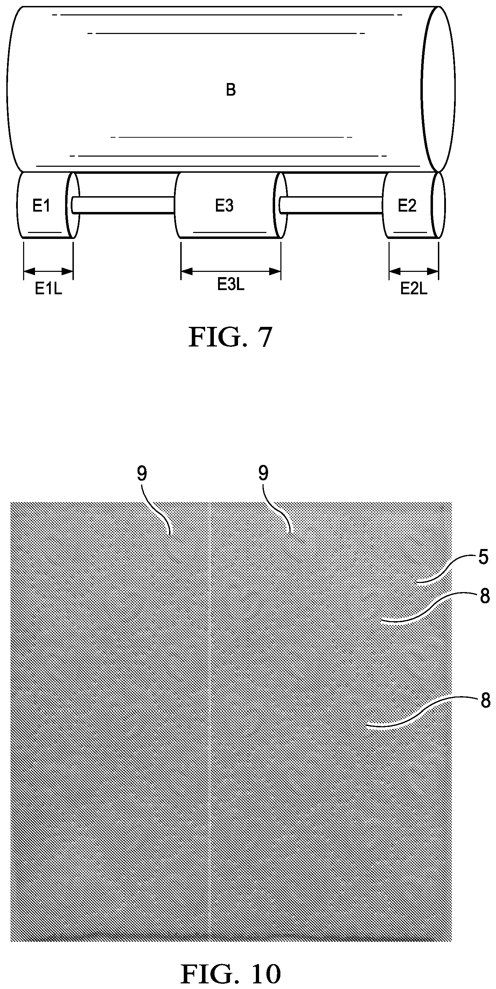

Fig. 7 is a simplified front view of another apparatus in Fig. 5 showing arrangement of roll B and nip rolls E. While Fig. 6 depicts an apparatus which may produce a single lane of three-dimensional substrate, Fig. 7 illustrates an example of apparatus designed to produce multiple lanes, two lanes according to the apparatus in Fig. 7, of three-dimensional substrates by increasing the number of nip rolls generating areas with increased peel force. Referring to Fig. 3 and Fig. 7, the length E1L of nip roll E1, and the length E2L of nib roll E2 determine a width W4 of the first area 332, and a width W5 of the second area 334, respectively. The length E3L of nip roll E3 may be a sum of W4 and W5.

Engagement of roll B and nip rolls E increases bonding strength between the first and second layers, and may have a negative impact on three-dimensional features such as protrusions as they are compressed by engagement of roll B and nip rolls E. Therefore, such a selective nip process can be designed to enable peel force increase only in pre-determined areas selectively while protecting the softness and visual quality of three-dimensional substrate in the rest.

When an absorbent article comprises the three-dimensional substrate disclosed herein, the three-dimensional substrate may be disposed to form at least part of the garment-facing side of the article in such a way that the first layer is oriented outwardly relative to an absorbent article, so that the protrusions can be felt by the caretaker or a user feeling the garment-facing side of the article. Or, the three-dimensional substrate may be disposed to form at least part of the skin-facing side of the article in such a way that the first layer is oriented outwardly relative to an absorbent article, so that the protrusions can be felt by wearers touching the skin-facing side of the article.

### Absorbent Article

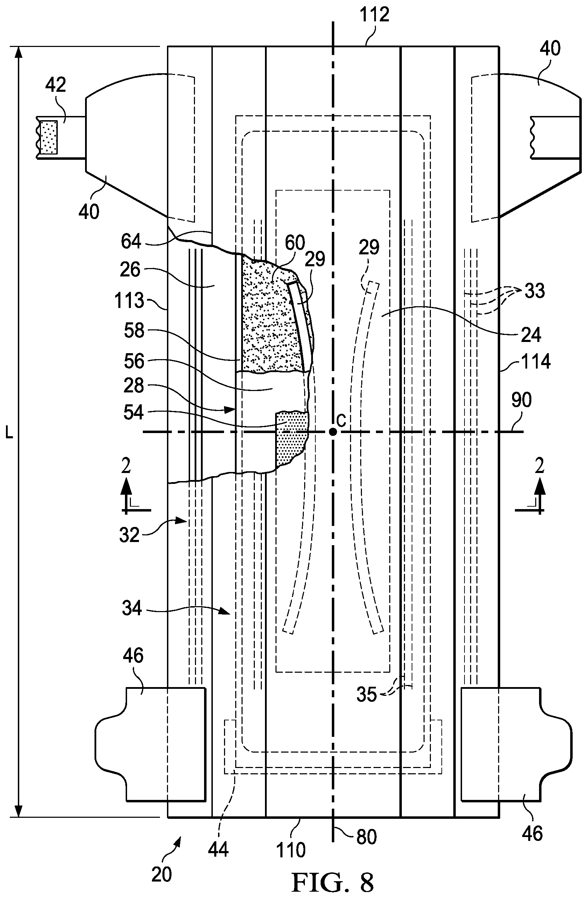

Absorbent articles will now be generally discussed and further illustrated in the form of a baby diaper 20 as exemplarily represented in Fig. 8. Fig. 8 is a plan view of the exemplary diaper 20 in a flattened-out configuration with the taped ends opened and the garment-facing side turned up. An article that is presented to the user closed such as a training pant may also be represented flattened out by cutting it along its side waists. The absorbent article will typically have a front edge 110, a back edge 112 and the longitudinally-extending lateral side edges 113, 114. The front edge 110 forms the edge of the front waist and the back edge 112 of the back waist, which together when worn by the wearer form the opening for the waist of the wearer. The lateral edges 113, 114 can each form one of the leg openings. The absorbent article 20 notionally comprises a longitudinal centerline 80 dividing the article in a left side and a right side, and a perpendicular transversal centerline 90 disposed at half the length of the article as measured on the longitudinal centerline 80, with both centerlines crossing at the center point C. The taped back ends 42 attached on the front of the diaper to such as a landing zone 44.

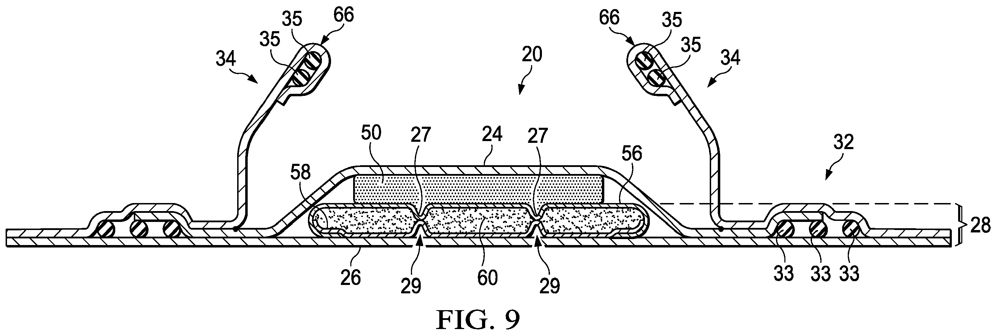

Other layers of the absorbent article are better illustrated in Fig. 9, which shows in cross-section in addition to the liquid permeable topsheet 24 and the backsheet 26, an absorbent core 28 between the topsheet 24 and the backsheet 26.

A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. An absorbent article according to the present invention may comprise a topsheet comprising a three-dimensional substrate disclosed herein so that a first layer of the three-dimensional substrate forms at least part of a wearer-facing surface of the absorbent article. In the absorbent article according to the present invention comprising a topsheet comprising a three-dimensional substrate disclosed herein, at least one of the first peel force and the second peel force is higher than a peel force between the three dimensional substrate and a layer such as an acquisition layer or a distribution layer disposed directly below the three-dimensional substrate.

An optional acquisition and/or distribution layer (or system) 50 is represented in Fig. 8 together with other typical diaper components. The acquisition and/or distribution layer may comprise one layer or more than one layer. Typical acquisition and/or distribution layers may not comprise SAP as this may slow the acquisition and distribution of the fluid, but an additional layer may also comprise SAP if some fluid retention properties are wished.

The absorbent article may typically comprise a pair of partially upstanding barrier leg cuffs 34 having elastic elements 35 and elasticized gasketing cuffs 32 having elastic elements 33 substantially planar with the chassis. Both types of cuffs are typically joined to the chassis of the absorbent article typically via bonding to the topsheet and/or backsheet.

The absorbent article may comprise elasticized back ears 40 having a tape end 42 which can be attached to a landing zone 44 at the front of the article, and front ears 46 typically present in such taped diapers to improve containment and attachment.

The absorbent article of the present invention may meet certain parametric requirements as detailed below. Among the parameters of interest, Thickness, Qmax (Qmax-dry and Qmax-wet) and Compression Recovery Rate ("CRR") are obtained from the FTT Test methods below. Information about the FTT Test method may be found in the paper "Fibers and Polymers 2014, Vol.15, No.7, 1548-1559" titled "A Simultaneous Measurement Method to Characterize Touch Properties of Textile Materials" by Xiao Liao et al.

### Absorbent core

As used herein, the term "absorbent core" refers to a component used or intended to be used in an absorbent article and which comprises an absorbent material and optionally a core wrap. As used herein, the term "absorbent core" does not include the topsheet, the backsheet and any acquisition-distribution layer or multilayer system, which is not integral part of the absorbent core. The absorbent core is typically the component of an absorbent article that has the most absorbent capacity of all the components of the absorbent article. The terms "absorbent core" and "core" are herein used interchangeably.

Referring to Figs. 5 and 6, the absorbent core 28 can absorb and contain liquid received by the absorbent article and comprise an absorbent material 60, which may be cellulose fibers, a blend of superabsorbent polymers and cellulose fibers, pure superabsorbent polymers, and/or a high internal phase emulsion foam. The absorbent core 28 may comprise absorbent material free channels 29, through which the top side 56 of the core wrap may be bonded to the bottom side 58 of the core wrap. The core wrap bonds 27 may at least persist as the absorbent core 28 swells upon liquid absorption and creates three-dimensional channels at the wearer-facing surface of the article. Of course, this is entirely optional, the absorbent core may also not have bonded channels, or even unbonded channels. The absorbent material defines an absorbent material area 8, which may be rectangular as show in in Fig. 8, but it is also common to have a shaped area which is tapered in the area around the transversal centerline 90.

The absorbent material comprises a liquid-absorbent material commonly used in disposable absorbent articles such as comminuted wood pulp, which is generally referred to as airfelt or fluff. Examples of other suitable liquid-absorbent materials include creped cellulose wadding; melt blown polymers, including co-form; chemically stiffened, modified or cross-linked cellulosic fibers; tissue, including tissue wraps and tissue laminates, absorbent foams, absorbent sponges, superabsorbent polymers (herein abbreviated as "SAP"), absorbent gelling materials, or any other known absorbent material or combinations of materials.

The absorbent material in the absorbent core can be any type. It can be an airfelt core comprising wood cellulose fibers such as pulp fibers mixed with SAP, or an airfelt-free core free from such cellulose fibers. Airfelt cores typically comprises from 40% to 80% of SAP. For absorbent cores comprising a relatively high proportion of SAP at least partially enclosed within the core wrap, the SAP content may represent in particular at least 80%, 85%, 90%, 95% and up to 100%, of superabsorbent polymer by weight of the absorbent material. The absorbent material may in particular comprise no or only small amount of cellulose fibers, such as less than 20%, in particular less than 10%, 5% or even 0% of cellulose fibers by weight of the absorbent material. The absorbent core may comprise an absorbent material comprising at least 80%, at least 90%, at least 95%, or at least 99% by weight of the absorbent core. The term "superabsorbent polymer" refers herein to absorbent material, which may be cross-linked polymer, and that can typically absorb at least 10 times their weight of an aqueous 0.9% saline solution as measured using the Centrifuge Retention Capacity (CRC) test (EDANA method WSP 241.2-05E). The SAP may in particular have a CRC value of more than 20 g/g, or more than 24 g/g, or of from 20 to 50 g/g, or from 20 to 40 g/g, or from 24 to 30 g/g. The SAP may be typically in particulate forms (superabsorbent polymer particles), but it not excluded that other forms of SAP may be used such as a superabsorbent polymer foam for example.

### Backsheet

Referring to FIG. 9, an absorbent article 20 according to the present invention comprises a backsheet 24. The backsheet may be designed to prevent the exudates absorbed by and contained within the absorbent article from soiling articles that may contact the absorbent article, such as bed sheets and undergarments. The backsheet may be substantially water-impermeable. Suitable backsheet materials may include breathable materials that permit vapors to escape from the absorbent article while still preventing exudates from passing through the backsheet. The backsheet may comprise a liquid impermeable film. The backsheet may comprise a wetness indicator. The backsheet may comprise a three-dimensional substrate disclosed herein.

### Outer Cover

An absorbent article 20 according to the present invention may comprise an outer cover forming at least part of a garment-facing surface of the absorbent article. The outer cover comprises a three-dimensional substrate disclosed herein so that a first layer of the three-dimensional substrate forms at least part of the garment-facing surface of the substrate. The three-dimensional substrate disclosed herein is disposed on the garment-facing side of the article. When a backsheet comprises a liquid impermeable polymer film, the polymer film and the substrate disclosed herein may be disposed in a face to face relationship in a such way that the substrate is towards the garment-facing side of the article, and the film is towards an absorbent core of the article. In that case, the first peel force of the region 302 and/or the second peel force of the second region 304 may be higher than a peel force between the three dimensional substrate and the film.

The absorbent article may also comprise other typical components, which are not represented, such as a back-elastic waist feature, a front elastic waist feature, transverse barrier cuff(s), a lotion application, etc.

Components of the disposable absorbent article described in this specification can at least partially be comprised of bio-sourced content as described in US 2007/0219521A1 Hird et al published on September 20, 2007, US 2011/0139658A1 Hird et al published on June 16, 2011, US 2011/0139657A1 Hird et al published on June 16, 2011, US 2011/0152812A1 Hird et al published on June 23, 2011, US 2011/0139662A1 Hird et al published on June 16, 2011, and US 2011/0139659A1 Hird et al published on June 16, 2011. These components include, but are not limited to, topsheet nonwovens, backsheet films, backsheet nonwovens, side panel nonwovens, barrier leg cuff nonwovens, super absorbent, nonwoven acquisition layers, core wrap nonwovens, adhesives, fastener hooks, and fastener landing zone nonwovens and film bases. In at least one embodiment, a disposable absorbent article component comprises a bio-based content value from about 10% to about 100% using ASTM D6866-10, method B, in another embodiment, from about 25% to about 75%, and in yet another embodiment, from about 50% to about 60% using ASTM D6866-10, method B. In order to apply the methodology of ASTM D6866-10 to determine the bio-based content of any disposable absorbent article component, a representative sample of the disposable absorbent article component must be obtained for testing. In at least one embodiment, the disposable absorbent article component can be ground into particulates less than about 20 mesh using known grinding methods (e.g., Wiley^{®} mill), and a representative sample of suitable mass taken from the randomly mixed particles.

### MEASUREMENT

### 1. Peel Force Test

### Sample preparation

The Peel Force Test is used to determine the Peel Force between the first layer and the second layer of a laminate material as described herein.

When a laminate specimen is available in its raw material form, a specimen of 50.8 mm (±0.2 mm) in wide (CD) and 200.0 mm (±0.2 mm) in length (MD) to include one of the first longitudinal edge 312 and the second longitudinal edge 314 referring to Fig. 2 is excised from the raw material using a precision cutter.

When a laminate specimen is obtained from an absorbent article, the laminate is first removed from the absorbent article using a precision cutter in a manner that avoids imparting any distortion or alteration of the bonds of the laminate during the process.

Using a precision cutter, referring to Fig. 2, a rectangle strip having a 50.8 mm width in MD direction and including one of the first longitudinal edge 312 and the second longitudinal edge 314 referring to Fig. 2 is cut from the absorbent article. Then, the laminate is removed from other components of the absorbent article. If needed to avoid any distortion or alteration of the bonds between layers of the laminate, a layer such as a film layer, directly attached to the laminate may remain while removing the laminate from the other components of the absorbent article. Then, a specimen of 50.8mm in wide (CD) and 200.0 mm (MD) in length including one of the first longitudinal edge 312 and the second longitudinal edge 314 is excised using a precise cutter to obtain a specimen for peel force measuring a peel force of the first region 302 or a peel force of the second region 304. A specimen for measuring a peel force of the third region 306 is prepared in the same way except cutting a rectangle strip having a 50.8 mm width in MD direction to include a longitudinal axis 308 from an absorbent article. Prepare 3 specimens in like fashion.

If the dimensions of the absorbent article do not allow to excise a specimen of 50.8 mm x 200.0 mm, the largest possible rectangular area will be excised from the absorbent article with the procedure above.

### Peel Force Measurement

A peel force between two layers in a laminate specimen is analyzed according to the National Standard of People's Republic of China standard method GB 8808-88 with the following specific choices and modifications:
- Testing speed Method A is used in which the crosshead speed is 300 mm/min;
- Specimen size: 50.8 ± 0.03mm in width and 200.0 ± 0.1 mm length. A length of a specimen corresponds to a machine direction of the specimen.
- A specimen is oriented in the tensile tester with the MD dimension of the specimen being oriented parallel to the direction of crosshead travel. To test a MD peel force, crosshead travel distance is set as 315mm.

The average peel force determined according to GB 8808-88 is defined as the Peel Force and is reported in units of force (Newtons) per specimen CD width (50.8 mm). This choice of units is expressed as "N/(50.8 mm)." In case the specimen width is different than 50.8 mm, the force is scaled appropriately and the result is reported in units of Newtons per 50.8 mm width (that is, N/(50.8mm)). Regardless, the force value in the numerator is reported to the nearest 0.01 N. Peel Force data corresponding to the first and last 15 mm in the portion of the peel data with force reading indicative of laminate peel are excluded from the average.

### EXAMPLES

### Example 1. Nonwoven Preparation

Nonwoven 1 as an example of three-dimensional substrates disclosed herein was prepared as below. A first layer material of 21gsm carded air-through bonded nonwoven comprising PE/PET fibers (50% 1.2dpf PE/PET and 50% 2dpf PE/PET fibers), and a second layer material of 21gsm carded air through nonwoven made of 2dpf PE/PET fibers were processed according to a process as illustrated in Figs. 5 and 6 or similar ones to obtain an apertured three-dimensional nonwoven as an example of the substrate disclosed herein with the pattern of Fig. 10. The pattern in Fig 10 has protrusions 9, apertures 5 and one or more lands 8. Nonwoven 1 has a first area 332 having a width W4 of 15mm, and a second area 334 having a width W5 of 15mm, referring to Fig. 3.

Nonwoven 2 as another example of three-dimensional substrates disclosed herein was produced by the same process as the process to producing nonwoven 1 except for using nip rolls having a width of 40mm to generate a first area 332 having a width W4 of 40mm, and a second area 334 having a width W5 of 40mm, referring to Fig. 3.

Nonwoven 3 was produced by the same process as the process producing nonwoven 1 except for not employing nip rolls to compress the side regions.

### Example 2. Nonwoven Peel Force Measurement

Peel forces of Nonwovens 1-3 were measured, according to the Peel Force Test described under MEASUREMENT above and illustrated in Table 1, respectively, below.

**Table 1**

| | | Nonwoven 1 (W4=W5= 15 mm) | Nonwoven 2 (W4=W5= 40mm) | Nonwoven 3 |
|---|---|---|---|---|
| Peel force (N/50.8mm) | First peel force | 0.47 | 1.13 | 0.12 |
| | Third peel force | 0.36 | 0.37 | 0.15 |
| | Second peel force | 0.51 | 1.2 | 0.12 |

### Example 3. Absorbent article Preparation

Diapers as exemplary absorbent articles containing an outer cover comprising a nonwoven layer were prepared using Pampers Hajimeteno Hadaeno Ichiban (Procter and Gamble Japan K.K. Japan) product design and nonwoven 1 and nonwoven 3 as outer most nonwoven layers, respectively.

### Example 4. Delamination Test

Diaper users having babies were provided Diapers 1, 2 and 3, and requested to report numbers of diapers they observed a delamination in the outer most nonwoven layer. Results are indicated in Table 2.

**Table 2**

| | Diaper 1* | Diaper 2* | Diaper 3 |
|---|---|---|---|
| Outer cover | Nonwoven 1 | Nonwoven 1 | Nonwoven 3 |
| No. of diapers tested | 1691 | 1699 | 304 |
| No. of diapers reported outer cover lamination | 7 | 5 | 5 |
| Delamination Rate (%) | 0.40 | 0.30 | 1.7 |

| | | | |
|---|---|---|---|
| *Diaper 1 and Diaper 2 only differ from each other in a belt material. | | | |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

While particular embodiments of the present invention have been illustrated and described, it would be obvious to those skilled in the art that various other changes and modifications can be made without departing from the scope of the invention as defined by the claims.

## Claims

1. A three-dimensional substrate (30) comprising a first layer (1), which is a nonwoven web of natural fibers, synthetic fibers or a combination of natural and synthetic fibers, and a second layer (2), which is a nonwoven web of natural fibers, synthetic fibers or a combination of natural and synthetic fibers, intermittently bonded to the first layer (1), and further comprising a first longitudinal edge (312) and a second longitudinal edge (314),
wherein the three-dimensional substrate (30) comprises a first region (302) longitudinally extending along the first longitudinal edge (312) and having a first peel force, a second region (304) longitudinally extending along the second longitudinal edge (314) and having a second peel force, and a third region (306) located between the first (302) and second regions (304) in a transversal direction and having a third peel force, as measured according to Peel force test of the description,
the substrate (30) has a transversal directional width of W perpendicular to a longitudinal direction L, and the first region (302), the second region (304), and the third region (306) have widths of W1, W2 and W3,
the third region (306) has a width W3 of at least about 30% of the substrate width W; and wherein at least one of the first peel force and the second peel force is higher than the third peel force;
wherein the first layer (1) comprises a plurality of protrusions (9) where the first layer (1) is unbonded to the second layer (2).

2. The three-dimensional substrate according to claim 1, wherein the first peel force and the second peel force are higher than about 0.15N/50.8mm as measured by the method of the description.

3. An absorbent article (20) having a wearer-facing surface and a garment-facing surface opposite to the wearer facing surface, comprising:
a liquid pervious topsheet (24),
a liquid impervious backsheet (26),
an absorbent core (28) disposed between the topsheet (24) and the backsheet (26), and
a three-dimensional substrate (30) according to any of the preceding claims.

4. The absorbent article according to claim 3, wherein the topsheet (24) comprises the three-dimensional substrate (30) wherein the first layer (1) forms at least part of the wearer-facing surface.

5. The absorbent article according to claim 4, wherein the first layer (1) comprises at least 80% of cotton fibers by weight of the first layer (1).

## Patentansprüche

1. Dreidimensionales Substrat (30), umfassend eine erste Schicht (1), die eine Vliesbahn aus Naturfasern, synthetischen Fasern oder einer Kombination aus Natur- und synthetischen Fasern ist, und eine zweite Schicht (2), die eine Vliesbahn aus Naturfasern, synthetischen Fasern oder einer Kombination aus Natur- und synthetischen Fasern ist, die mit der ersten Schicht (1) unterbrochen gebunden ist, und ferner umfassend einen ersten Längsrand (312) und einen zweiten Längsrand (314),
wobei das dreidimensionale Substrat (30) einen ersten Bereich (302), der sich in Längsrichtung entlang der ersten Längsrands (312) erstreckt und eine erste Schälkraft aufweist, einen zweiten Bereich (304), der sich in Längsrichtung entlang des zweiten Längsrands (314) erstreckt und eine zweite Schälkraft aufweist, und einen dritten Bereich (306), der sich in Querrichtung zwischen dem ersten (302) und dem zweiten Bereich (304) befindet und eine dritte Schälkraft aufweist, gemessen nach der Schälkraftprüfung der Beschreibung, umfasst, wobei das Substrat (30) eine Breite W in Querrichtung lotrecht zu einer Längsrichtung L aufweist und der erste Bereich (302), der zweite Bereich (304) und der dritte Bereich (306) Breiten von W1, W2 und W3 aufweisen,
wobei der dritte Bereich (306) eine Breite W3 von wenigstens etwa 30 % der Substratbreite W aufweist; und wobei wenigstens eine der ersten Schälkraft und der zweiten Schälkraft höher als die dritte Schälkraft ist;
wobei die erste Schicht (1) eine Vielzahl von Vorsprüngen (9) umfasst, wobei die erste Schicht (1) mit der zweiten Schicht (2) ungebunden ist.

2. Dreidimensionales Substrat nach Anspruch 1, wobei die erste Schälkraft und die zweite Schälkraft höher als etwa 0,15 N/50,8 mm sind, gemessen durch das Verfahren der Beschreibung.

3. Absorptionsartikel (20), der eine trägerseitig Oberfläche und eine bekleidungsseitige Oberfläche entgegengesetzt zu der trägerseitigen Oberfläche aufweist, umfassend:
eine flüssigkeitsdurchlässige Oberschicht (24),
eine flüssigkeitsundurchlässige Unterschicht (26),
einen Absorptionskern (28), der zwischen der Oberschicht (24) und der Unterschicht (26) angeordnet ist und
ein dreidimensionales Substrat (30) nach einem der vorstehenden Ansprüche.

4. Absorptionsartikel nach Anspruch 3, wobei die Oberschicht (24) das dreidimensionale Substrat (30) umfasst, wobei die erste Schicht (1) wenigstens einen Teil der dem trägerseitigen Oberfläche ausbildet.

5. Absorptionsartikel nach Anspruch 4, wobei die erste Schicht (1) wenigstens zu 80 % Baumwollfasern umfasst, bezogen auf das Gewicht der ersten Schicht (1).

## Revendications

1. Substrat tridimensionnel (30) comprenant une première couche (1), qui est un voile non tissé de fibres naturelles, de fibres synthétiques ou d'une combinaison de fibres naturelles et synthétiques, et une seconde couche (2), qui est un voile non tissé de fibres naturelles, de fibres synthétiques ou d'une combinaison de fibres naturelles et synthétiques, collé par intermittence à la première couche (1), et comprenant en outre un premier bord longitudinal (312) et un second bord longitudinal (314),
dans lequel le substrat tridimensionnel (30) comprend une première région (302) s'étendant longitudinalement le long du premier bord longitudinal (312) et ayant une première force de pelage, une deuxième région (304) s'étendant longitudinalement le long du second bord longitudinal (314) et ayant une deuxième force de pelage, et une troisième région (306) située entre la première (302) et la deuxième région (304) dans une direction transversale et ayant une troisième force de pelage, comme mesurée selon le test de force de pelage de la description, le substrat (30) a une largeur transversale directionnelle de W perpendiculaire à une direction longitudinale L, et la première région (302), la deuxième région (304) et la troisième région (306) ont des largeurs de W1, W2 et W3,
la troisième région (306) a une largeur W3 d'au moins 30 % environ de la largeur W de substrat ; et dans lequel au moins l'une de la première force de pelage et de la deuxième force de pelage est supérieure à la troisième force de pelage ;
dans lequel la première couche (1) comprend une pluralité de protubérances (9) où la première couche (1) n'est pas liée à la seconde couche (2).

2. Substrat tridimensionnel selon la revendication 1, dans lequel la première force de pelage et la deuxième force de pelage sont supérieures à environ 0,15 N/50,8 mm comme mesuré par le procédé de la description.

3. Article absorbant (20) ayant une surface orientée vers le porteur et une surface orientée vers le vêtement opposée à la surface orientée vers le porteur, comprenant :
une feuille de dessus perméable aux liquides (24),
une feuille de fond imperméable aux liquides (26) ;
une âme absorbante (28) disposée entre la feuille de dessus (24) et la feuille de fond (26), et
un substrat tridimensionnel (30) selon l'une quelconque des revendications précédentes.

4. Article absorbant selon la revendication 3, dans lequel la feuille supérieure (24) comprend le substrat tridimensionnel (30), dans lequel la première couche (1) forme au moins une partie de la surface orientée vers l'utilisateur.

5. Article absorbant selon la revendication 4, dans lequel la première couche (1) comprend au moins 80 % de fibres de coton en poids de la première couche (1).
